# EUROPEAN PATENT APPLICATION

(11) **EP 1 978 013 A1**
(43) Date of publication of application: **08.10.2008**
(21) Application number: 07007018.0
(22) Date of filing: 04.04.2007
(51) Int. Cl.: C07C 69/675, C07C 69/68, C07C 69/704, C07C 69/70, C07C 69/732, C07C 69/34, C07C 67/08, C07C 67/26, C08G 63/06, C08G 63/16, C08G 18/34

(54) **Diols and polyols**

(71) Applicant: Cognis IP Management GmbH, 40589 Düsseldorf (DE)
(72) Inventor: Bigorra Llosas, Joaquin, 08201 Sabadell (ES); Llaurado, Luis, 08440 Cardedeu (Barcelona) (ES); Gusi, Francesc, 08028 Barcelona (ES)
(74) Representative: Fabry, Bernd

(57) **Abstract**

Suggested are new diols and polyols encompassing hydroxypropyl esters of hydroxycarboxylic or polycarboxylic acids. The diols and polyols are useful as monomer compounds and reactive solvents.

## Description

### Field of the invention

The present invention relates to the area of polymers and refers to new reactive diols and polyols, respectively, based on hydroxycarboxylic acids, a process for obtaining them, and their use as monomers and reactive solvents.

### Background of the invention

A diol or glycol is a chemical compound containing two hydroxyl groups. Vicinal diols have hydroxyl groups attached to adjacent atoms. Examples of vicinal diol compounds are ethylene glycol and propylene glycol. Geminal diols have hydroxyl groups which are bonded to the same atom. Diols represent important intermediates, especially for the production of polymers such as polyesters and polyurethanes:

**R-COOH + HO-[Y]-OH + HOOC-R → R-CO-O-[Y]-O-OC-R + 2 H₂O**

**R-N=C=O + HO-[Y]-OH → R-NH-CO-O-[Y]-O-OC-NH-R**

Quite a number of diols are used as monomers for making polyesters or polyurethanes: typical examples encompass glycols, especially ethylene glycol and propylene glycol. In case it is desirable to have molecules with a longer chain length than the hydrogenation products of dicarboxylic acid, for example, 1,6-hexanediol or 1,12-dodecandiol are rather useful. A third group of diols comprises the ring-opening products of epoxidised olefins, for example, the ring opening product of 1,2-epoxydodecane with water.

A serious disadvantage, however, is that diols according to the state of the art usually exhibit either an unsatisfying polarity or lack of a chemical structure which is either too short-chained or disadvantageous for other reasons. For example, glycols such as ethylene glycol or propylene glycol, as well as the longer chained α,ω-diols, are rather suitable for the production of symmetrical polymers, however, the polarity of the diols becomes the poorer the longer the carbon chain is. On the other hand, ring-opening products of epoxides exhibit a much better polarity due to vicinal OH groups, however they do not allow to produce symmetrical polymers. Although it is theoretically possible to synthesise tailor-made diols meeting all requirements with regard to polarity and structure, the state of the art suggests only rather complex and cost-intensive solutions which are not suitable to be reduced into practice.

A second problem relates to the fact that polymers are usually sold as solutions or dispersions, which require specific organic solvents. Suitable solvents encompass aromatic compounds, alcohols, and ketones, which all share the same disadvantage of being highly volatile. Solvents with high VOC, however, become more and more restricted for environmental reasons.

Therefore, the object of the present invention has been to find one solution for two very different problems for the polymer industry. The first problem underlying the invention has been to develop new diols with improved polarity and high flexibility with respect to their chemical structure which are obtainable with little technical afford. The second problem relates to the identification of new solvents for polymer solutions or dispersions, which allows production of compositions with high solids matter at low viscosity while the VOC is reduced at the same time.

### Detailed description of the invention

The present invention refers to new diols or polyols encompassing monopropylenglycol esters of hydroxycarboxylic acids or monopropylenglycol esters of polycarboxylic acids, and more particularly
(i) Glycolic acid hydroxypropyl ester,
(ii) Lactic acid hydroxypropyl ester,
(iii) (Iso)Citric acid mono/di/tri hydroxypropyl ester,
(iv) Malic acid mono/di hydroxypropyl ester,
(v) Tartaric acid mono/di hydroxypropyl ester,
(vi) Ricinoleic acid hydroxypropyl ester,
(vii) 12-Hydroxystearic acid hydroxypropyl ester,
(viii) Diols according to general formula (I) 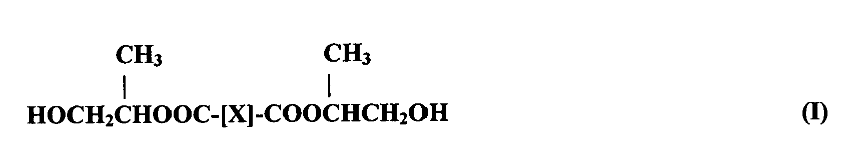 in which [X] represents an akylene or alkenylene radical having 2 to 12 carbon atoms or an aromatic ring system of 6 carbon atoms.

Surprisingly it has been observed that hydroxypropyl esters of hydroxycarboxylic acids solve the complex problem in an ideal way. The esterification of hydroxycarboxylic acids with propylene glycol or by reaction with propylene oxide leads to the formation of diols or polyols depending on the number of carboxylic acid functions in the molecule. The -COO- function near the terminal OH group improves the polarity of the molecule, while the preferred chemical structure of the diol or polyol can be selected in a wide range by choosing the adequate hydroxycarboxylic acid or polycarboxylic acid, in particular dicarboxylic acid. The esterification can be conducted according to known manners and represents a standard unit process within the chemical industry. At the same time, the new diols exhibit also very good solvent properties for a wide range of polymers, e.g. polyesters, polyurethanes, or poly(meth)-acrylates. Using the new products as solvents allows to increase the amount of solids in the composition, while viscosity is maintained, or to decrease viscosity while maintaining the solids amount - depending on the kind of product the market requires. Due to the free hydroxyl groups in these new solvents they can react with isocyanates together with the hydroxyl groups of the resins, reducing the amount in VOC.

### Esterification

Another object of the present invention relates to a process for the production of diols and/or polyols, which is characterised in that hydroxycarboxylic or polycarboxylic acids are esterified with propylene glycol according to known manners or by reaction of the carboxylic groups with on average 1 mole of propylene oxide. Suitable hydroxycarboxylic acids are selected from the group consisting of glycolic acid, lactic acid, (iso)citric acid, malic acid, tartaric acid, ricinoleic acid, 12-hydroxystearic acid and their mixtures. As an alternative, saturated or unsaturated, aliphatic or aromatic dicarboxylic acids can also be chosen, like adipic acid, maleic acid 1,12-dodecanoic acid, or phthalic acid. In these cases the diols are obtained by esterification of both carboxylic acid functions with propylene glycol.

It is, however, also possible to choose other materials not mentioned here, which are obtainable by specific synthesis, for example, by epoxidation of oleic acid and subsequent ring opening by means of water or alcohols. Also aromatic acids can be used such as, e.g., gallic acid.

Esterification can take place according to the methods known in organic chemistry. Usually one places the hydroxycarboxylic acid (or the dicarboxylic acid) and the propylene glycol in a molar relation of 1:1 to 1:1.2 in order to ensure a complete reaction. In case two- or three-basic carboxylic acids are used, the molar relation depends on whether it is wished to generate a mono or higher ester; nevertheless, the same rules apply. The condensation reaction is conducted at elevated temperatures of about 80 to about 120 °C, while the reaction water is continuously separated off in order to move the equilibrium of the reaction towards the esterification products. Although 1.3-propyleneglycol represents the usual starting product, one can also use 1,2-propyleneglycol or even mixtures. It is also possible to add propylene oxide to the hydroxycarboxylic acids, however, in this case one must either make sure that the free OH group is protected against the attack of the epoxide, or accept that at least a part of the hydroxyl groups becomes etherified.

### Industrial application

Another object of the present invention refers to the use of the new hydroxypropyl esters as monomer or co-monomer components in the production of polyesters and polyurethanes.

A final object of the invention is directed to the use of the new diols or polyols as reactive solvents for polymers, especially for polyesters, polyurethanes and poly(meth)acrylates. The compositions thus obtained typically contain solids amounts of 30 to 80, preferably 35 to 65 % b.w. - the remainder being the new diol or polyol, preferably lactic acid hydroxypropylester.

### Examples

### Example 1

### Lactic acid hydroxypropylester

In a 5-1-flask fitted with stirrer and water remover 900 g (10 moles) lactic acid, 1,330 g (17.5 moles) propyleneglycole, 50 g of an acid ion exchanger resin (Amberlite^{®} IRA 118) and 11 chloroform were placed. The mixture was heated under reflux until the condensation of water had come to an end. Subsequently, the mixture was cooled down, the resin was removed, and the solvent along with non-reacted alcohol was separated from the remaining ester.

### Example 2

### Tartaric acid mono/dihydroxypropylester

In a 5-1-flask fitted with stirrer and water remover 1,500 g (10 moles) tartaric acid, 1,520 g (20 moles) propyleneglycole, 70 g of an acid ion exchanger resin (Amberlite^{®} IRA 118) and 11 chloroform were placed. The mixture was heated under reflux until the condensation of water had come to an end. Subsequently, the mixture was cooled down, the resin was removed, and the solvent along with non-reacted alcohol was separated from the remaining mono/diester mixture.

### Example 3

### Citric acid mono/di/trihydroxypropylester

In a 5-1-flask fitted with stirrer and water remover 19,20 g (10 moles) citric acid, 2,230 g (20 moles) propyleneglycole, 90 g of an acid ion exchanger resin (Amberlite^{®} IRA 118) and 1 I chloroform were placed. The mixture was heated under reflux until the condensation of water had come to an end. Subsequently, the mixture was cooled down, the resin was removed, and the solvent along with non-reacted alcohol was separated from the remaining mono/di/triester mixture.

### Example 4

### Ricinoleic acid hydroxypropylester

A 2-1-pressure reactor was charged with 873 g (2.8 moles) ricinoleic acid and 7 g sodium tert.-butylate. The reactor was purged several times with nitrogen to remove all traces of oxygen and then heated to 125 °C. Subsequently, 62 g (1.1 moles) propylene oxide was added at a pressure of 2 bar. Once the acid number had decreased to less than 5, the reaction was stopped by cooling the reactor. The ricinoleic acid hydroxypropylester was received in the form of a clear liquid showing an average degree of propoxylation of 0.95.

## Claims

1. Hydroxypropylesters of hydroxycarboxylic acids or polycarboyxlic acids.

2. Glycolic acid hydroxypropyl ester

3. Lactic acid hydroxypropyl ester.

4. (Iso)Citric acid mono/di/tri hydroxypropyl ester.

5. Malic acid mono/di hydroxypropyl ester.

6. Tartaric acid mono/di hydroxypropyl ester.

7. Ricinoleic acid hydroxypropyl ester.

8. 12-Hydroxystearic acid hydroxypropyl ester.

9. Diols according to general formula (I) 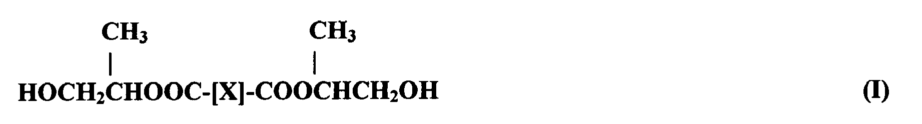 in which [X] represents an akylene or alkenylene radical having 2 to 12 carbon atoms or an aromatic ring system of 6 carbon atoms.

10. Process for the production of diols and/or polyols according to any of Claims 1 to 9, **characterised in that** hydroxy- or polycarboxylic acids are esterified with propylene glycol or by reaction with propylenoxyde according to known manners.

11. Use of the hydroxypropyl esters according to any of Claims 1 to 9 as monomer or co-monomer components in the production of polyesters and polyurethanes.

12. Use of the hydroxypropyl esters according to any of Claims 1 to 9 as reactive solvents for polymers.
